# FASCICULE DE BREVET EUROPEEN

(11) **EP 0 423 300 B1**
(45) Date de publication et mention de la délivrance du brevet: **04.01.1995**
(21) Numéro de dépôt: 90907136.7
(22) Date de dépôt: 12.04.1990
(51) Int. Cl.: C07B 37/12, C07H 15/10, C07C 45/69

(54) **MILIEU REACTIONNEL CHIRAL POUR DES REACTIONS ORGANIQUES**
CHIRALES REAKTIONSMEDIUM FÜR ORGANISCHE REAKTIONEN
CHIRAL REACTION MEDIUM FOR ORGANIC REACTIONS

(30) Priorité: 14.04.1989 FR 8904950
(43) Date de publication de la demande: 24.04.1991
(73) Titulaire: ERIDANIA BEGHIN-SAY, F-59239 Thumeries (FR)
(72) Inventeur: LUBINEAU, André, F-91410 Dourdan (FR); BIENAYME, Hugues, F-75014 Paris (FR); QUENEAU, Yves, F-92100 Boulogne-Billancourt (FR)
(74) Mandataire: David, Daniel
(86) Numéro de dépôt international: FR9000267
(87) Numéro de publication internationale: WO9012773

(56) Documents cités:
- US-A- 4 144 261
- Journal of the American Chemical Society, volume 104, No. 21, 20 October 1982, American Chemical Society, pages 5853-5854
- Journal of the American Chemical Society, volume 102, No.26, 17 December 1980, American Chemical Society, D.C. Rideout et al, pages 7816-7817.
- The Journal of Organic Chemistry, Volume 52, No. 6, 20 March 1987, American Chemical Society, A. Lubineau et al, pages 1001-1007.
- The Journal of Organic Chemistry, volume, 48, No. 18, 9 September 1983, American Chemical Society, P.A. Grieco et al, Pages 3137-3139.

## Description

La présente invention concerne un nouveau milieu réactionnel chiral pour des réactions organiques.

Il existe un besoin industriel général d'optimisation des réactions organiques et des recherches sont constamment menées dans ce but. De nombreux efforts ont porté particulièrement sur l'augmentation de la vitesse des réactions. Une telle augmentation de la vitesse réactionnelle n'a le plus souvent pu être obtenue qu'au prix de l'utilisation de catalyseur(s) et/ou de pressions élevées, ce qui constitue des mesures coûteuses et/ou difficiles à mettre en oeuvre. Dans de nombreux cas, l'emploi de solvants organiques a aussi été préconisé, le cas échéant en association avec d'autres mesures telles que celles énoncées précédemment, mais outre le fait que le coût de tels solvants est en général élevé, voire prohibitif, leur utilisation entraîne souvent des problèmes liés à leur toxicité.

Il existe également un besoin industriel général de pouvoir modifier la sélectivité de réactions organiques, notamment la régiosélectivité, la stéréosélectivité, l'énantiosélectivité et la chimiosélectivité. Diverses solutions ont été proposées à cet égard, en fonction du problème particulier à résoudre, parmi lesquelles on peut citer l'utilisation de solvants chiraux mais le coût de ces derniers est si élevé que des procédés mettant en jeu des réactions effectuées dans de tels solvants sont peu intéressants du point de vue économique.

La présente invention fournit un milieu réactionnel pouvant notamment permettre d'augmenter sensiblement la vitesse de réactions organiques et de modifier le cas échéant leur sélectivité tout en palliant les difficultés énoncées ci-dessus.

La présente invention a pour objet l'utilisation d'une solution aqueuse concentrée d'au moins un hydrate de carbone choisi parmi les mono-, di- et trisaccharides et leurs alkyl-glycosides dont le groupement alkyle a de 1 à 4 atomes de carbone, comme milieu réactionnel chiral pour des réactions organiques.

A titre d'exemples non limitatifs d'hydrates de carbone utilisables dans la présente invention on peut citer le glucose, le fructose, le saccharose, le ribose, le galactose, le mannose, l'α-méthyl glucoside

Par "solution concentrée" il faut entendre une solution dont la concentration est au moins égale à environ 0,2 M mais cette valeur n'est pas critique et la limite inférieure de la concentration en ledit hydrate de carbone sera aisément déterminée par l'homme de l'art pour la réaction spécifique considérée.

La présente invention sera à présent décrite en se référant plus particulièrement aux réactions de Diels-Alder mais il faut comprendre que l'exemple constitué par ce type de réaction n'est donné que pour illustrer à la fois ce caractère inattendu de l'invention et certains avantages qu'elle est susceptible de procurer et qu'il ne doit en aucun cas constituer une limitation de sa portée.

Il est connu que des réactions de Diels-Alder sont considérablement accélérées dans l'eau, comparativement à des solvants organiques (voir Rideout, D.C., Breslow, R. J. Am. Chem. Soc., 1980, 102, 7816 ; Grieco, P.A. ; Yoshida, K., Garner, P. J. Org. Chem., 1983, 48, 3139 et Lubineau, A., Queneau, Y. J. Org. Chem., 1987, 52, 1001.

Il est également connu que la cyclodextrine β accélère des réactions de Diels-Alder en milieu aqueux par formation d'un amas hydrophobe du diène et du diènophile à l'intérieur de sa cage hydrophobe (voir Rideout, D.C., Breslow, R. J. Am. Chem. Soc., 1980, 102, 7816, déjà cité et Sternbach, D.D., Rossana, D.M. J. Am. Chem. Soc., 1982, 104, 5853).

Les cyclodextrines sont des hydrates de carbone mais, contrairement aux mono-, di- et trisaccharides utilisés dans la présente invention, elles sont constituées de 6, 7 ou 8 unités de glucose formant un anneau comportant en son centre une cage de nature hydrophobe.

Les solutions aqueuses de mono-, di- et trisaccharides n'étant pas connues pour former de telles cages, on pourrait s'attendre à ce que l'accélération dans de tels milieux soit très inférieure à celle obtenue avec un milieu réactionnel constitué par une solution saturée de ladite cyclodextrine.

D'une façon surprenante, la demanderesse a constaté non seulement que des solutions aqueuses d'hydrates de carbone choisis parmi les mono-, di- et trisaccharides et leurs alkyl-glycosides dont le groupement alkyle a de 1 à 4 atomes de carbone constituent un milieu réactionnel permettant d'augmenter la vitesse de réactions organiques mais encore que l'accélération ainsi obtenue peut même être sensiblement supérieure à celle procurée par une solution aqueuse saturée d'une cyclodextrine, comme cela est montré dans les Exemples non limitatifs ci-après.

Le milieu réactionnel chiral de la présente invention peut être utilisé par exemple pour effectuer toutes les cycloadditions, notamment les réactions de Diels-Alder, les cycloadditions photochimiques, en photochimie par exemple pour réaliser des réarrangements photo-Smiles, des inductions asymétriques dans les réactions de réduction, des fonctionnalisations régiosélectives de composés aromatiques (hydroxyméthylation, chloration, iodation, etc.), des condensations aldoliques, des époxydations asymétriques, des hydrolyses d'esters avec des chaînes lipophiles, dans la chimie organométallique des éléments de transition, pour l'oxydation des oléfines (notamment par le procédé Wacker), etc.

### Exemples

Les Exemples 1 à 3 ci-dessous concernent la réaction de Diels-Alder entre le 1-β-D-glucosyl-1,3-butadiène et la buténone à 25°C sous pression atmosphérique dans des milieux réactionnels aqueux. Le 1-β-D-glucosyl-1,3-butadiène de formule 1 ci-dessous : 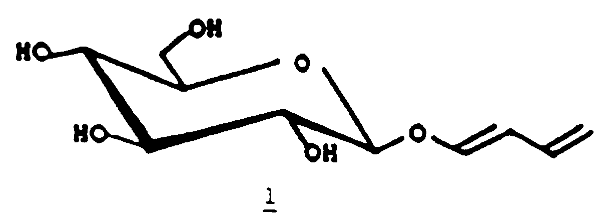
a été préparé comme indiqué dans la publication précitée Lubineau. A., Queneau, Y. J. Org. Chem., 1987, 52, 1001.
Nous rappelons ci-dessous ce mode de préparation.

### Préparation du 1-β-D-glucosyl-1,3-butadiène (diène 1)

On a traité (a) de l'acétobromoglucose 2 (que l'on peut se procurer aisément) dans une solution de Me₂SO par le sel de sodium de malonaldéhyde. On a obtenu l'aldéhyde 3 que l'on a isolé par cristallisation directe avec un rendement de 56 %. La réaction est la suivante : 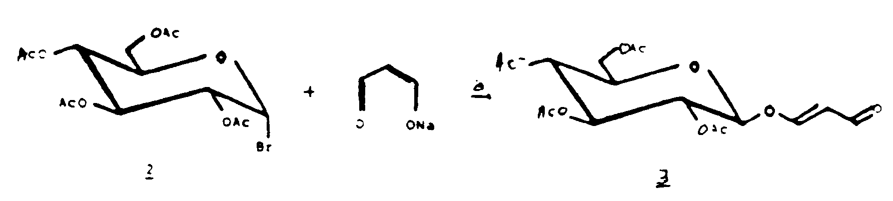
Conformément à la publication David, S. ; Lubineau, A. ; Vatèle, J.M., Nouv. J. Chim. 1980, 4, 547, l'addition (b) de méthylènetriphénylphosphorane "exempt de sel" à l'aldéhyde 3 à - 78°C dans un mélange oxolane-toluène conduit au diène 4 de configuration β avec un rendement de 83 %. La désacétylation (c) dans un mélange NET₃-MeOH-H₂O (1:8:1) conduit au diène 1 avec un rendement quasi quantitatif. La réaction est la suivante :

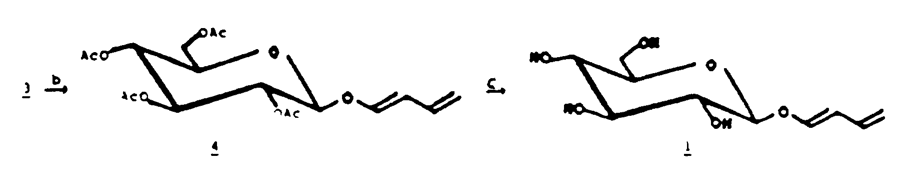

### Exemple 1

Dans cet Exemple on a effectué la réaction de Diels-Alder entre le 1-β-D-glucosyl-1,3-butadiène (1) et la buténone (5) (réaction (I)) 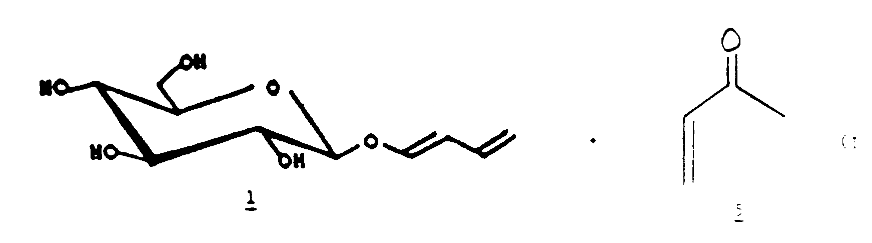
à 25°C et sous pression atmosphérique, dans de l'eau pure (essai comparatif 1), dans une solution aqueuse de MeOH à 50 % (essai comparatif 2), dans des solutions aqueuses de glucose 0,5M, de glucose 1M, de glucose 2M, glucose 3M, de saccharose 0,5M, de ribose 2M, de mannose 1M, de galactose 1M, (respectivement, essais 3 à 10 selon la présente invention), de cyclodextrine β saturée, c'est-à-dire 0,19 M en unité glucose (essai comparatif 11) et d'α-méthylglucoside 0,5M, 2M et 2,5M (respectivement essais 12 à 14 selon la présente invention).

La cyclodextrine β a été obtenue auprès de la Société Janssen Chimica.

Le Tableau I ci-dessous indique les valeurs de la constante de vitesse du deuxième ordre k x 10⁵ M⁻¹s⁻¹ pour chacun des essais pour la réaction considérée à 25°C avec chacun des additifs cités.

**TABLEAU I**

| ESSAI | ADDITIF | kx10⁵M⁻¹s⁻¹ |
|---|---|---|
| 1 (comparatif) | Aucun (H₂O) | 28,2 |
| 2 (comparatif) | MeOH 50 % | 7,5 |
| 3 | Glucose 0,5M | 29,8 |
| 4 | Glucose 1M | 34,8 |
| 5 | Glucose 2M | 44,6 |
| 6 | Glucose 3M | 61,3 |
| 7 | Saccharose 0,5M | 32 |
| 8 | Ribose 2M | 35,1 |
| 9 | Mannose 1M | 32,5 |
| 10 | Galactose 1M | 34,2 |
| 11 (comparatif) | Cyclodextrine β solution saturée 0,19 M en unité glucose | 41,6 |
| 12 | α-méthylglucoside 0,5M | 30,9 |
| 13 | α-méthylglucoside 2M | 43,7 |
| 14 | α-méthylglucoside 2,5M | 48,1 |

Comme on le peut voir dans le Tableau I, par rapport à l'eau pure, la réaction est ralentie par la présence d'alcool simple (méthanol) alors qu'elle est accélérée par les solutions aqueuses d'hydrates de carbone.

Comme on peut également le voir d'après le Tableau I (comparaison des essais 5, 6 et 11) d'une façon surprenante, le glucose déjà à la concentration 2M apporte une accélération sensiblement plus importante qu'une solution saturée de cyclodextrine β connue pourtant pour accélérer les réactions de Diels-Alder par formation d'un amas hydrophobe du diène et du diènophile dans sa cage hydrophobe.

Le glucose à la concentration 3M apporte un gain de vitesse de 117 % par rapport à l'eau pure et de 47 % par rapport à une solution saturée de cyclodextrine β.

### Exemple 2

La réaction de Diels-Alder entre le 1-β-D-glucosyl-1,3-butadiène et la buténone a été effectuée à 25°C sous pression atmosphérique dans des milieux réactionnels constitués par des solutions aqueuses contenant de 0 à 10 mol % de glucose afin de déterminer la variation de la constante de vitesse du deuxième ordre en fonction de la concentration de glucose. Aucun effet d'accélération mesurable n'a été décelé pour des milieux réactionnels constitués par des solutions aqueuses de glucose jusqu'à 0,2 M.

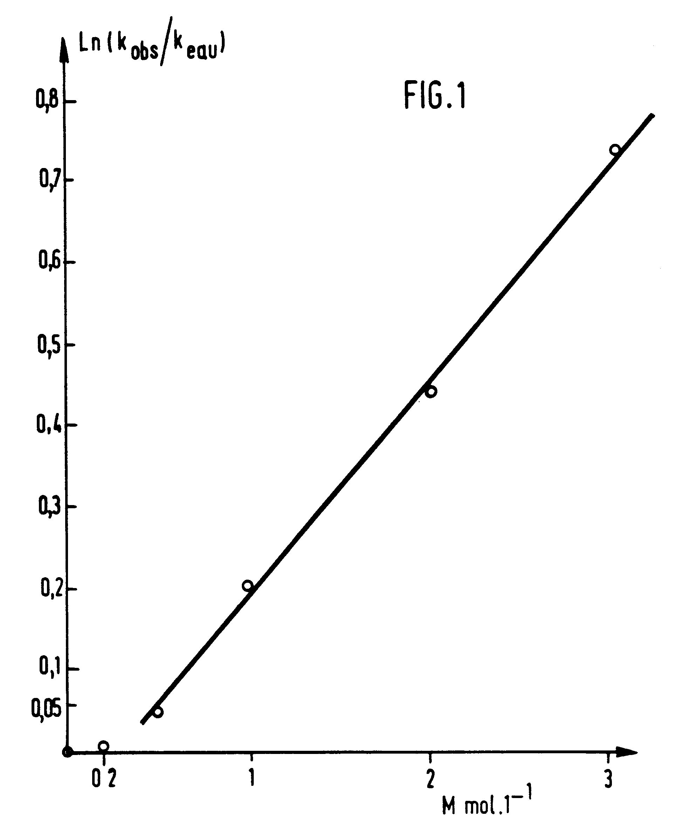

La Figure 1 annexée représente la courbe de variation de Ln k_{obs}/kₑₐᵤ en fonction de la concentration en glucose dans l'intervalle 0,5M-3M ; cette courbe est une droite.

Pour des concentrations très élevées en glucose (3,5 M), on n'a observé aucune augmentation de l'accélération de la réaction par rapport à une solution 3M.

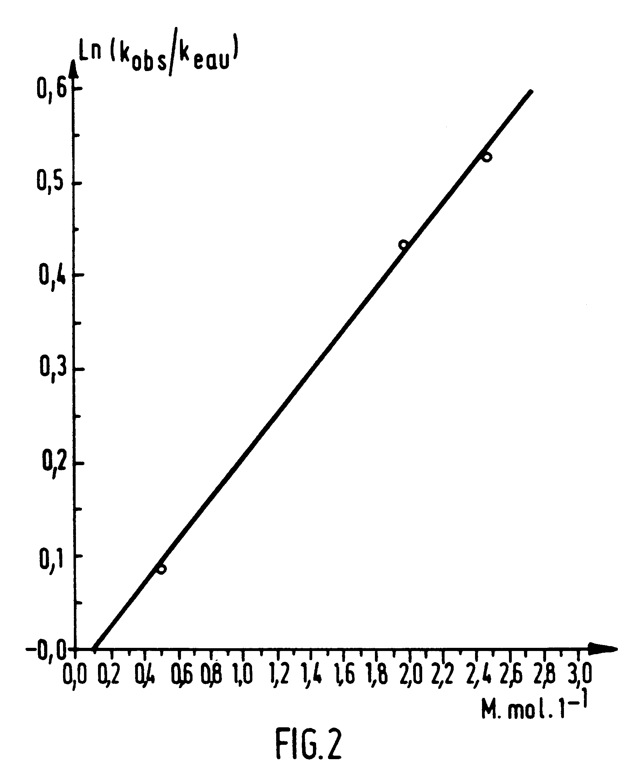

La Figure 2 annexée représente la variation Ln(k_{obs}/kₑₐᵤ) en fonction de la concentration en α-méthyl glucoside ; cette courbe est une droite.

### Exemple 3 (comparatif)

Afin de déterminer l'éventuelle influence de la viscosité de la solution de glucose sur la vitesse de la réaction considérée (à 25°C, sous pression atmosphérique), on a mesuré les constantes de vitesse du deuxième ordre dans une solution de glucose 2,6m et dans des solutions de polyéthylèneglycol (PEG 6000 obtenu auprès de la Société Janssen Chimica) de viscosité similaire et de viscosité supérieure à celle de ladite solution de glucose. Les résultats sont donnés dans le Tableau II ci-dessous.

**TABLEAU II**

| | H₂O | Glucose (2,6 m) | PEG 6000 (70 g/l) | PEG 6000 (140 g/l) |
|---|---|---|---|---|
| Viscosité (cPo) | 0,9426 | 2,845 | 2,527 | 5,507 |
| kx10⁵M⁻¹s⁻¹ | 28,2 | 44,9 | 25,5 | 23 |

Comme on peut le voir d'après le Tableau II, le PEG 6000, contrairement au glucose, retarde la réaction et l'effet de décélération est même d'autant plus important que la viscosité augmente.

### Exemple 4

Dans cet Exemple on a effectué la réaction de Diels-Alder entre le méthoxybutadiène (6) et la buténone (5) (réaction (II)) 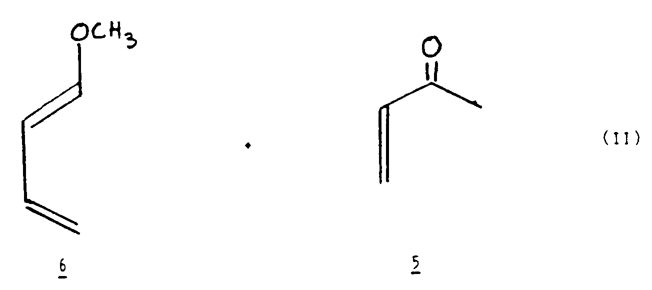
à 25°C et sous pression atmosphérique dans de l'eau pure (essai comparatif 15), dans une solution aqueuse de MeOH à 50% (essai comparatif 16), dans des solutions de glucose 0,5M de glucose 1M, de glucose 1,5M, de glucose 4M, de saccharose 0,5M, de saccharose 1M, de saccharose 2M, de ribose 1M, de ribose 2M, de ribose 3M (respectivement, essais 17 à 26, selon la présente invention) et de cyclodextrine β saturée, c'est-à-dire 0,19M en unité glucose (essai comparatif 27).

La cyclodextrine β a été obtenue auprès de la Société Janssen Chimica.

Le Tableau III ci-dessous indique les valeurs de la constante de vitesse du deuxième ordre k x 10⁵ M⁻¹ s⁻¹ pour chacun des essais pour la réaction considérée avec chacun des additifs cités.

**TABLEAU III**

| ESSAI | ADDITIF | kx10⁵M⁻¹s⁻¹ |
|---|---|---|
| 15 (comparatif) | Aucun (H₂O) | 109 |
| 16 | MeOH 50 % | 23,4 |
| 17 | Glucose 0,5M | 117 |
| 18 | Glucose 1M | 133 |
| 19 | Glucose 1,5M | 154 |
| 20 | Glucose 4M | 300 |
| 21 | Saccharose 0,5M | 123 |
| 22 | Saccharose 1M | 152 |
| 23 | Saccharose 2M | 230 |
| 24 | Ribose 1M | 124 |
| 25 | Ribose 2M | 137 |
| 26 | Ribose 3M | 166 |
| 27 (comparatif) | Cyclodextrine β solution saturée 0,19 M en unité glucose | 254 |

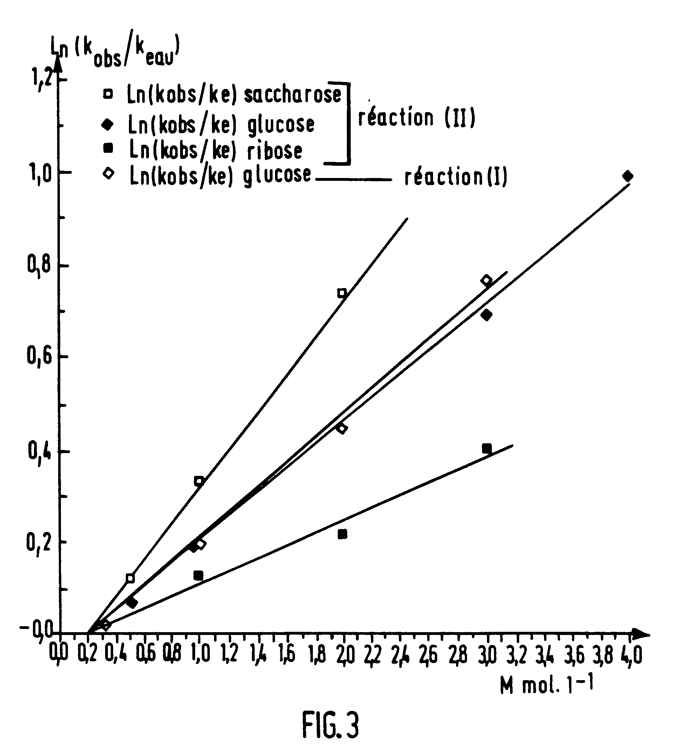

La Figure 3 annexée représente la variation Ln kobs/keau en fonction de la concentration en saccharose, glucose, ribose pour la réaction (II) du présent Exemple 4 ainsi qu'en fonction du glucose pour la réaction (I) de l'Exemple 1 ci-dessus.

La comparaison entre l'Exemple 1 et l'Exemple 4 montre que tous les hydrates de carbone revendiqués augmentent la vitesse de réaction alors que le méthanol la diminue.

On remarquera que l'effet du glucose est similaire sur les deux réactions (I) et (II) (les droites représentant Ln k_{obs}/kₑₐᵤ en fonction de la concentration en glucose pour chacune de ces réactions sont pratiquement confondues).

On notera également qu'aussi bien dans la réaction I que dans la réaction II, le glucose concentré apporte une accélération sensiblement plus importante qu'une solution de cyclodextrine saturée.

On observe également qu'en molarité le saccharose augmente plus la vitesse de réaction que le glucose, lui-même plus que le ribose.

Les Exemples ci-dessus illustrent certaines performances du nouveau milieu réactionnel chiral de l'invention qui permet notamment d'obtenir une augmentation considérable de la vitesse de réactions organiques de façon simple et économique et sans entraîner de problèmes de toxicité.

## Revendications

1. Utilisation d'une solution aqueuse concentrée d'au moins un hydrate de carbone choisi parmi les mono-, di- et trisaccharides et leurs alkyl-glycosides dont le groupement alkyle a de 1 à 4 atomes de carbone, comme milieu réactionnel chiral pour des réactions organiques.

2. Utilisation selon la revendication 1, caractérisée en ce que ledit hydrate de carbone est du glucose.

## Claims

1. Use of a concentrated aqueous solution of at least one carbohydrate selected from the mono-, di- and trisaccharides and their alkyl glycosides of which the alkyl group has 1 to 4 carbon atoms, as a chiral reaction medium for organic reactions.

2. Use according to Claim 1, characterized in that the said carbohydrate is glucose.

## Patentansprüche

1. Verwendung einer konzentrierten wäßrigen Lösung von zumindest einem Kohlehydrat gewählt aus den Mono-, Di- und Trisacchariden und ihren Alkyl-Glykosiden, deren Alkylgruppe 1 bis 4 Kohlenstoffatome hat, als chirales Reaktionsmedium für organische Reaktionen.

2. Verwendung gemäß Anspruch 1, dadurch gekennzeichnet, daß das Kohlehydrat Glucose ist.
